Europäisches Patentamt

European Patent Office

Office européen des brevets

(19)

(11) Veröffentlichungsnummer: **0 489 332 A1**

# (12) EUROPÄISCHE PATENTANMELDUNG

(21) Anmeldenummer: **91120111.9**

(22) Anmeldetag: **26.11.91**

(51) Int. Cl.⁵: **C12N 5/00**, C12N 5/06

(30) Priorität: **01.12.90 DE 4038397**

(43) Veröffentlichungstag der Anmeldung:
**10.06.92 Patentblatt 92/24**

(84) Benannte Vertragsstaaten:
**AT BE CH DE DK ES FR GB GR IT LI LU NL SE**

(71) Anmelder: **BOEHRINGER INGELHEIM KG**

**W-6507 Ingelheim am Rhein(DE)**

(84) **BE CH DE DK ES FR GR IT LI LU NL SE AT**

(71) Anmelder: **BOEHRINGER INGELHEIM INTERNATIONAL G.M.B.H.**

**W-6507 Ingelheim am Rhein(DE)**

(84) **GB**

(72) Erfinder: **Kutsch, Horst, Priv. Doz. Dr. habil.
Dillinger Strasse 27
W-6690 St. Wendel 1(DE)**

(54) **Mikrocarrier für verankerungsbedürftige Zellen.**

(57) Die Erfindung betrifft einen Mikrocarrier aus ausschließlich biologischem Material für lebende Zellkulturen.

EP 0 489 332 A1

Die Erfindung betrifft ein modifiziertes Trägermaterial als Mikrocarrier für verankerungsbedürfte Zellen, insbesondere für Säugerzellen.

Nach dem Stand der Technik war es bekannt, für verankerungsbedürftige Zellen Träger mit Glasoder Kunststoffoberflächen zu verwenden. Das Scale-up wird dabei durch einfaches Vergrößern der Oberflächen erreicht.

Seit 1967 werden alternativ Mikrocarrier zur Anzucht und Kultur von verankerungsbedürftigen Zellen eingesetzt. Als Trägermaterial dienen verschiedene Materialien (Glas, Kunststoffe, vernetzte Polysaccharide). Die Adhäsion wurde durch chemische Modifikation der Trägeroberflächen entscheidend verbessert. In den meisten Fällen wird dazu Collagen kovalent an die Oberflächen der Träger gebunden. Das Scale-up im Falle von Mikrocarriern erfolgt durch einfache Zugabe frischen Trägermaterials; dabei erfolgt eine Zellwanderung von bereits besiedelten Carriern hin zu den frischen Trägern.

Erfindungsgemäß wird eine neue Klasse von derivatisierten vesikulären Materialien (ZSM = zellstrukturiertes Material) zur Verwendung als Trägermaterial vorgeschlagen, die aus hochgereinigten ganzen Zellen bzw. Zellverbänden von Pflanzen bestehen.

Die übrigbleibenden Zellwände bestehen dabei nur noch aus natürlichen Polysacchariden, was mit entscheidenden Vorteilen verbunden ist. Der leere Innenraum (Vesikel) kann Flüssigkeiten und darin gelöste Substanzen aufnehmen.

Die Herstellung und allgemeine Verwendung solchen Materials als chromatographisches Medium wurde bereits dem DD-Patent 247 570 offenbart.

Ein solches Material kann im Gegensatz zu den konventionellen Materialien als voll-biologisch bezeichnet werden. Während die Oberflächen konventioneller Trägermaterialien mehr oder weniger regelmäßig geformt sind, stellen die Oberflächen von vesikulären Materialien geometrisch komplexe, quasi-natürliche fraktale Gebilde dar, wodurch die Adhäsion von Zellen begünstigt wird.

Das erfindungsgemäße modifizierte Trägermaterial kann nach folgendem Verfahren hergestellt werden: Trockenes vesikuläres Material wird auf eine Partikelgröße (Korngröße) von 20 bis 300 $\mu$m gesiebt; bevorzugt ist eine Partikelgröße zwischen 60 und 125 $\mu$m. Das abgesiebte Material wird mit einer 10 - 50 millimolaren NaIO$_4$-Lösung - deren pH-Wert sauer eingestellt ist - versetzt, deren Menge so abgemessen ist, daß das getrocknete vesikuläre Material darin quellen kann. Man läßt das Material zwischen 30 und 180 Minuten in dieser Lösung oxidieren und wäscht es anschließend mehrmals mit Wasser - möglichst destilliertem Wasser - aus. Bevorzugtes Oxidationsmittel ist eine 0.15 molare Phosphat-Pufferlösung (pH = 4,5), die 20 mMol/ltr NaIO$_4$ enthält, wobei das vesikuläre Material 60 Minuten mit dieser Lösung behandelt wird.

Das so behandelte und gewaschene Material wird dann in verdünnter wässeriger Proteinlösung über Nacht inkubiert, wobei die Konzentration der Proteinlösung wie auch die Temperatur der Proteinlösung je nach Protein einzustellen ist. Hinsichtlich der Temperaturbedingungen sollte Raumtemperatur (ca. 20° C) nicht überschritten werden. Für Collagen erwies sich eine 1 Gew.-%-ige Lösung in Wasser bei einer Behandlungstemperatur von 4° C als günstig. Anschließend wird das mit dem Protein beschichtete vesikuläre Material weiterverarbeitet, in dem es in einer wässerigen Lösung von Glycin oder Glycinamid für ca. 1 Stunde inkubiert wird, z.B. in einer 0.5 molaren wässerigen Glycinlösung. Danach kann die Hydrierung der Azomethin-Bindungen mit einem geeigneten Reduktionsmittel in wässerigem Medium, z.B. mit Natriumborhydrid (NaBH$_4$, 0,1 %ig in wässerigem Puffer) für ca. 20 Minuten bei 4° C erfolgen. Bevorzugt ist ein PBS-Puffer, 1 molar, unter Zugabe von trockenem Puffermaterial zur Konstanthaltung des pH-Wertes. Anschließend wird das Material wieder gut mit Wasser gewaschen, der nun fertige Mikrocarrier wird in wässeriger 0.9 Gew.-% NaCl-Lösung suspendiert. Ein weiterer wesentlicher Vorteil des erfindungsgemäßen Mikrocarriers ist es , daß er ausschließlich aus biologischem Material besteht und daß seine Herstellung im Vergleich zu den bisher bekannten Carriern für lebende Zellen einfacher und kostenkünstiger ist. Erfindungsgemäß wird ein solches vesikuläres Material so modifiziert, daß eine die Adhäsion fördernde Substanz kovalent auf der fraktalen Oberfläche des vesikulären Materials gebunden ist. Solche adhäsionsfördernden Stoffe sind bekannt, so z.B. Proteine und Peptide insbesondere Collagen, wie z.B. Collagen R oder natives Collagen, Laminin und vi greenforms Der erfindungsgemäße Mikrocarrier kann als Trägermaterial für verankerungsbedürftige, lebende Zellen verwendet werden. Die Präparation derartiger Systeme mit lebenden Zellen sind aus dem Stand der Technik bekannt.

Aufgrund der Materialeigenschaften von ZSM erfolgt die Sterilisierung bevorzugt durch Gamma-Bestrahlung (Dosis 5 - 10 kGy). Zur Vermeidung von strahlungsbedingten Schäden am Carrier (Radikalreaktionen) können der Suspensionslösung 0.1 % oder mehr Histidin oder Histidinamid oder andere Radikalfänger zugesetzt werden.

Anstelle von kovalent gebundenen Collagen kann der erfindungsgemäße Mikrocarrier auch andere Proteine oder Peptide als Adhäsiosfaktoren kovalent gebunden enthalten. Beispielhaft seien genannt:

- Laminin (Glycoprotein, Mr ca. $10^6$; für Epidermal- u. Endothelialzellen).
- poly-D-Lysin (Mr 30 - 70.000 bzw. 70-150.000).

Tierische Zellen, die beispielsweise auf dem erfindundungsgemäßen Mikrocarrier wachsen können, sind:

- V79 Hamsterlungen-Fibroblasten,
- Humanendothelialzellen,
- Hamsternierenzellen,
- Goldhamsterovarialzellen,

Die nachfolgenden Beispiele sollen die Erfindung erläutern, ohne sie in ihrem Umfang einzuschränken.

Präparation des vesikulären Trägermatrials (Beispiele)

Hier: Zuckerrüben-Material

1. Zellaggregate aus der Zellkultur mit Leitungswasser gut waschen.

2. Phosphorsaures EtOH (1 ml Phosphorsäure pro 1 l 80 - 90 % EtOH) in die Packung aus Zellaggregaten hineinwaschen. Für eine Packung von 3 - 4 l werden ca. 7 l angesäuerten EtOH benötigt. EtOH wird am Ablauf zur Regeneration aufgefangen, sobald der ablaufende Extrakt klar und stark gelb gefärbt ist. Die Extraktion wird danach mit 96 % EtOH fortgesetzt, bis die Zellaggregate entfärbt sind und der Extrakt farblos abläuft (ca. 6 l).

3. Die EtOH-feuchte Packung wird nun mit 6 l 1 % Phosphorsäure (mit $Na_2HPO_4$ auf pH 2.5 gebracht) gespült. Die Spülung ist beendet, wenn der ablaufende EtOH einen Wassergehalt von ca. 40 % aufweist.

4. Anschließend wird die Packung mit je 3 l Aqua dest. zweimal aufgerührt und unter Rühren bis zum Festwerden drainiert. Anschließend wird mit 3 l Aqua dest. im Festbett eluiert.

5. Die Packung wird unter Zugabe von Aqua dest. auf ein Suspensionsvolumen von ca. 6 l gebracht. Die Suspension wird nun auf pH 6.5 gebracht und mit 7 g KCl versetzt. In die Suspension werden 0.3 l Trypsinlösung (ca. 23 g/l) eingerührt.

6. Ansatz 24 Stunden unter langsamem Rühren auf 20 - 25 °C halten. Danach nochmals 0.3 l Trypsinlösung wie unter (5) zugeben.

7. Nach ca. 48 Stunden Zugabe von 5 ml eines neutral reagierenden ionischen Tensids (Konzentrat). Nachspülen mit Aqua dest.

8. Die wasserfeuchte Packung wird nun mit ca. 6 l 0.5 % NaCl-Lösung gewaschen. Packung danach in 3 l Soerensen-Puffer (pH 7) suspendieren und der Puffer ablaufen lassen. In die Packung nun 3 l Aqua dest. einziehen lassen.

9. Der Ansatz nach (8) kann nun einer Naßsiebung zugeführt werden oder nach Trocknung im Luftstrom einer Trockensiebung zugeführt werden.

Präparation des vesikulären Trägermaterials (Beispiel)

Hier: Kohl-Material

1. Gewaschener Kohl wird entblättert und gehäckselt. Danach gut mit Leitungswasser waschen.

2. 100 - 120 kg gehäckselte Masse werden in angesäuertem Wasser (400 l $H_2O$ + 1 l $H_3PO_4$ + 0.2 kg $Na_2HPO_4$) inkubiert (24 - 48 h, 4-10 °C). Ablauf und Waschen mit EtOH (50 %, dann 70 %).

3. Nach Ablauf der wässrigen EtOH-Ablauge wird mit ca. 60 °C heißem 80 -90 % EtOH extrahiert. Dabei werden Lipidbestandteile herausgelöst und Wasser verdrängt.

4. Pflanzenmasse mit Wasser waschen. Nach Ablauf des Waschwassers Masse in 100 Liter Trypsinlösung (enthält ca. 0.04 kg Trypsin) suspendieren und ca. 48 - 60 Stunden bei 20 - 25 °C langsam rühren. Die Suspensionslösung sollte einen pH von 6.5 aufweisen.

5. Nach Ablauf der Suspensionslösung wird die Packung unter Rühren mehrmals gewaschen, bis der Ablauf Ninhydrin-negativ ist.

6. Die Masse wird nun vermoost, nochmals gewaschen (Wasser) und mit EtOH (Gradient 50 - 70 - 80 - 90 - 96 %) entwässert.

7. Die Trocknung erfolgt im warmem Luftstrom. Anschließend kann das Trockenmaterial vermahlen werden.

1. Entfärbung und Lipidextraktion

1.1 Die Zellaggregate werden im Siebgefäß gesammelt und mit Leitungswasser gewaschen (dabei ohne Unterdruck arbeiten).

1.2 Die Zellaggregate werden mit phosphorsaurem Ethanol (1 ml Phorphorsäure pro 1 80 - 90 %iges Ethanol) entfärbt. Dazu wird das angesäuerte Ethanol zunächst zweimal in kleinen Mengen (weniger als 10 % des Packungsvolumens) auf die Packung gegeben. Bevor das erste Mal Ethanol zugegeben wird, sollte das Wasser noch in geringer Menge über der Packung stehen, damit anfangs ein Ethanolgradient durch die Packung läuft. Nachdem etwa 0,5 l sauren Ethanols in die Packung eingedrungen sind, wird das Material mit 6 l des angesäuerten Ethanols überschichtet. Es wird ohne Unterdruck im Festbett extrahiert. Das Ethanol wird am Ablauf zur Regeneration aufgefangen, sobald der ablaufende Extrakt klar und stark gelb gefärbt ist. Die Extraktion wird danach mit 96 %igem Ethanol fortgesetzt, bis die Zellen entfärbt sind und der Extrakt farblos abläuft (ca. 6 l).

## 2. Enzymatische Protoplasma-Solubilisierung und Extraktion (bezogen auf 3-4 l Packungsvolumen)

2.1 Die ethanolhaltige Packung wird mit 6 l 1%igen Phosphorsäurepuffers gespült. Die erforderliche Menge wird bereitet, indem 1%ige Phosphorsäure (techn.) bei potentiometrischer Kontrolle des pH mit gesättigter $Na_2HPO_4$-Lösung auf pH 2,5 gebracht wird. Das ablaufende Ethanol wird aufgefangen. Es wird ohne Unterdruck gearbeitet. Zunächst wird eine geringe Menge des Puffers in die Packungsoberfläche gerührt, solange etwas Ethanol über der Packung steht. Bei diesem Vorgang kommt es darauf an, Klumpenbildung an der Oberfläche zu vermeiden. Nach dem Festwerden der Packung wird 2x mit je 3 l des Phosphorsäurepuffers überschichtet, und das Ethanol wird gesammelt, bis es einen Wassergehalt von 40 % aufweist (Spindel).

2.2 Anschließend wird die Packung mit je 3 l Aqua dest. zweimal aufgerührt und unter Rühren bis zum Festwerden drainiert, anschließend mit 3 l Aqua dest. im Festbett eluiert.

2.3 Die feuchte Masse wird in ein 10 l-Gefäß überführt. Am Siebgefäß anhaftende Reste werden mit dest. Wasser in das gleiche Gefäß gespült. Durch Zugabe von Aqua dest. wird das Valumen der Suspension auf ca. 6 l gebracht. Bei potentiometrischer Kontrolle wird die Suspension mit gesättigter $Na_2HPO_4$-Lösung auf pH 6,5 gebracht, danach werden 7 g KCl und 3 g $NaN_3$ in die Suspension gerührt. 7 g Trypsin zur Zellzucht (Firma Belger, Kleinmachnow) werden in 0,3 l Aqua dest gut verrührt und in die Suspension der Zellaggregate gemischt. Das Gefäß wird abgedeckt und bei Zimmertemperatur (20 - 25°C) aufgestellt. Nach 24 h werden weitere 7 g Trypsin in oben beschriebener Weise zugegeben.

2.4. Nach etwa 48 h werden die Zellaggregate gewaschen. Hierzu gibt man 5 ml eines konzentrierten neutral reagierenden Spülmittels (Natriumalkylsulfonat) zu der Suspension, überführt sie vollständig mit Nachspülen (Aqua dest.) in das Siebgefäß, läßt die Masse absetzen und verrührt sie zweimal mit 3 l 0.5 %iger NaCl-Lösung. Anschließend wird die Masse abgedeckt und als geschlossene Packung mit 6 l NaCl-Lösung (0,5 %) gewaschen. Hieran schließt sich ein Aufrühren mit 3 l 0,1 M Phosphatpuffer pH 7 (Soerensen) an. Nach dem Festwerden läßt man 3 l Aqua dest. einziehen und benetzt anschließend die Oberfläche mit Ethanol. Beim Einziehen des Ethanols wird die Packung an der Oberfläche leicht verrührt und anschließend mit 80 - 90 %igem Ethanol (1.5 l) überschichtet. Nach dem Einziehen des 80 - 90 %igen Ethanols wird 96 %iges Ethanol (4 l) zugegeben. Das Ethanol läuft ohne Unterdruck durch die Packung. Das durchlaufende Ethanol wird gesammelt, sobald es eine Konzentration von > 60 % erreicht hat.

## 3. Trocknen

Die ethanolhaltige Masse wird auf einer großen Filternutsche durch den Unterdruck einer Wasserstrahlpumpe vom beweglichen Teil des Ethanols befreit. Dabei wird die Masse mit einem Plastspatel an den Rand der Nutsche gepreßt. Sie wird anschließend mit 1 l einer Mischung aus 96 %igem Ethanol und n-Butanol überschichtet (Verhältnis 10/1). Nach dem Einziehen der Ethanol/Butanol-Mischung wird die Packung trockengesaugt, wobei das Pulver an der Oberfläche gelockert und an den Rand der Nutsche gepreßt wird, damit das Alkoholgemisch gleichmäßig abgesaugt werden kann. Der in der Saugflasche gesammelte Alkohol wird zur Destillation gesammelt. Das gut abgesaugte, alkoholfechte Pulver wird im Vakuumrotationsverdampfer getrocknet. Hierbei ist darauf zu achten, daß sich im Rundkolben eine Schlaufe aus elastischem Material (z.B. Polyamidseil) befindet, damit das Pulver gut bewegt wird und sich keine Klumpen bilden können. Klumpenbildung kann durch vorheriges Sieben des feuchten Pulvers vollständig vermieden werden. Der Kolben des Rotationsverdampfers wird mit einem Wasserbad erwärmt (Wassertemperatur 70°C). Der Rundkolben ist zu einem Drittel mit dem Material zu füllen. Am Kolbenhals muß sich ein Filter aus Polyamidseide befinden, dessen Aufgabe darin besteht, das Heraussaugen des trockenen Pulvers aus dem Rundkolben zu verhindern.

## Präparation eines Derivats des vesikulären Trägermaterials aus Zuckerrüben

Hier: Collagen-Derivat (Beispiel)

1. Naßgesiebte Zellaggregate (150 - 300 $\mu$m) in Periodat-Lösung suspendieren (50 mM $NaIO_4$ + 500 ml MERCK-Puffer pH 4 + 2000 ml Aqua dest., R.T.). Auf 2.5 l der Periodat-Lösung sollten nicht mehr als ca. 1 kg wasserfeuchte Zellmasse kommen.

2. Ansatz bei R.T. und im Dunkeln ca. 1 Stunde langsam rühren.

3. Ansatz abfiltrieren und gut mit Aqua dest. waschen.

4. Die Feuchtmasse in ca. 1 l Collagen-Lösung (0.3-1%) inkubieren und über Nacht bei 4°C langsam rühren.

5. Danach gut mit Aqua dest. waschen.

6. 2.5 g NaBH₄ in 1.5 l MERCK-Puffer pH 7 lösen. In frische Lösung die Pflanzenzellmasse einrühren und ca. 30 Minuten bei ca. 10°C langsam weiterrühren. Anfangs-pH ca. 8, End-pH ca. 9.

7. Ansatz gut mit Aqua dest. waschen und fertiges Material wasserfeucht in 0.9 % NaCl-Lösung suspendieren. Danach Bestrahlung (Gamma, ca. 7 kGy).

Beispiel für die Kultivierung von lebenden Zellen auf dem erfindungsgemäßen Carriermaterial:

Zellinien: CHO-KI (ATCC CCL 61); BHK 21 (c-13) (ATCC CCL 10)

Die oben genannten Zellinien wurden ausgewählt, weil sie als Ausgangszellinien vieler genetisch modifizierter Zellinien zur Herstellung von zahlreichen rekombinaten Produkten wie AT III, Faktor VIII, Interleukine und Interferone dienen. In der Regel unterscheiden sich diese Ausgangszellinien hinsichtlich ihrer Anheftungseigenschaften nicht sehr von den genetisch modifizierten Produktionszellinien. Die mit den Ausgangszellinien ermittelten Daten lassen sich daher auf die meisten Produktionszellinien übertragen.

Versuche in Spinnerflaschen:
Carrierkonzentration:
63 g l⁻¹ Zuckerrüben-ZSM-Collagen-Gel,
Reaktionsvolumen: 100 bzw. 500 ml
Drehzahl: 40 Upm
Temperatur: 37°C, Brutschrank mit 7.5 % $CO_2$, Luft und 95 % Luftfeuchtigkeit
pH: 7.0 - 6.6
Medium: Dulbecco's Modified Eagel Medium (DMEM) 80 vol%, Tryptose-phosphate-broth 10 vol% und Fötales Kälberserum (FCS) 10 vol% Animpfkonzentration:
2 x 10⁵ Zellen ml⁻¹
Versuche im Fermenter:
Carrierkonzentration: 63 g l⁻¹
Zuckerrüben-ZSM-Collagen-Gel
Drehzahl: 45 Upm
Temperatur: 37°C
pH: 6.9
$pO_2$: 40 % Luftsättigung
Medium: Dulbecco's Modified Eagel Medium (DMEM) 80 vol%, Tryptose-phosphate-broth 10 vol% und Fötales Kälberserum (FCS) 10 vol%
Reaktionsvolumen: 2.2 l

Analytik:

Glukose und Laktat wurden enzymatisch mit einem Analyser der Firma YSI bestimmt. Die freien Zellen, dienicht auf dem Carrier gebunden sind, wurden direkt im Haemocytometer gezählt. Für die carriergebundenen Zellen wurden die Zellen bei den Vesipormaterialien mit Trypsin/EDTA-Lösung von den Trägern abgelöst und dann im Haemocytometer gezählt.

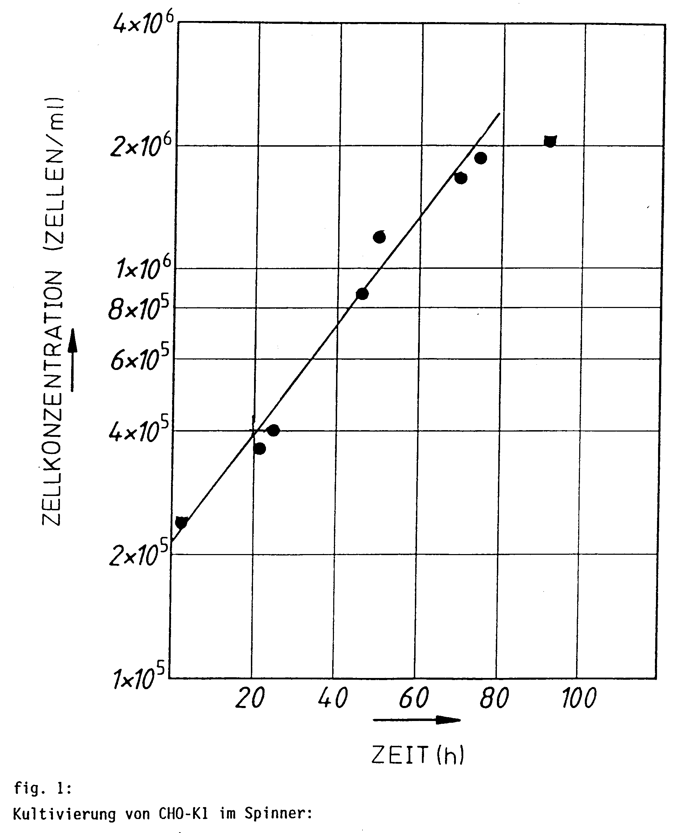

Figur 1 zeigt das Wachstumsverhalten von CHO-KI-Zellen auf Zuckerrüben-ZSM-Collagen.

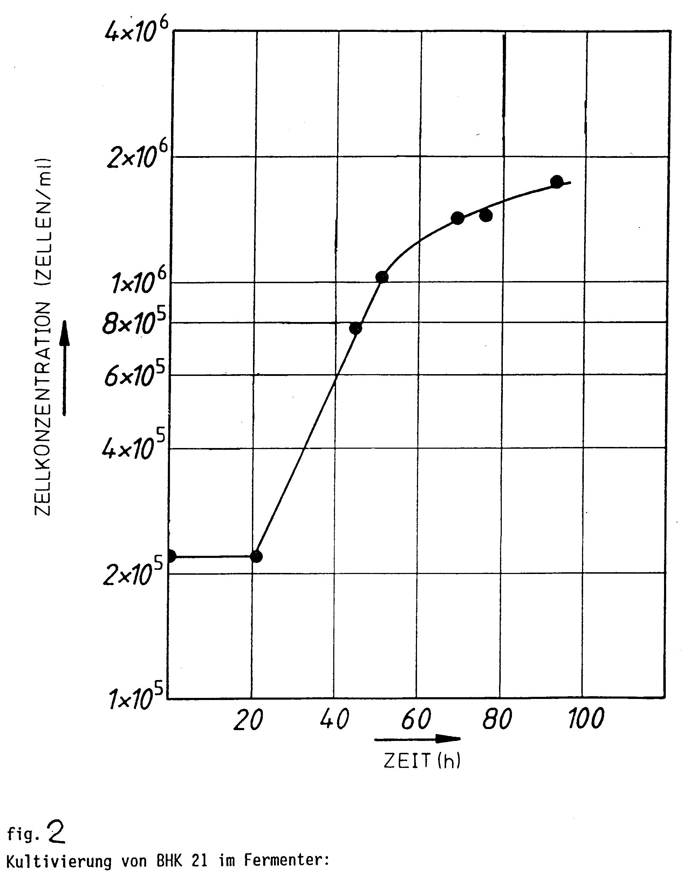

Figur 2 zeigt das Wachstumsverhalten von BHK 21 Zellen im Fermenter, ebenfalls auf Zuckerrüben-ZSM-Collagen.

**Patentansprüche**

1. Mikrocarrier für lebende Zellen, dadurch gekennzeichnet, daß er ein vesikuläres Material mit mindestens einer adhäsionsfördernden Substanz enthält.

2. Mikrocarrier nach Anspruch 1, dadurch gekennzeichnet, daß das vesikuläre Material eine Partikelgröße (trocken) zwischen 20 und 300 µm sowie eine fraktale Oberfläche (gequollener Zustand) aufweist.

3. Mikrocarrier nach Anspruch 1 oder 2, dadurch gekennzeichnet, daß die adhäsionsfördernde Substanz ein Protein oder Peptid ist.

4. Mikrocarrier nach Anspruch 3, dadurch gekennzeichnet, daß die adhäsionsfördernde Sustanz ein Collagen, Laminin oder poly-D-Lysin ist.

5. Verwendung von vesikulärem Material zur Herstellung von Mikrocarriern für lebende Zellen.

6. Verfahren zur Herstellung von Mikrocarriern für lebende Zellen, dadurch gekennzeichnet, daß ein getrocknetes vesikulares Material mit einer für lebende Zellen adhäsionsfördernden Substanz kovalent verknüpft wird.

fig. 1:
Kultivierung von CHO-K1 im Spinner:

fig. 2
Kultivierung von BHK 21 im Fermenter:

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | ABSTRACTS OF PAPERS  AMERICAN CHEMICAL SOCIETY,192 NO ACS NATIONAL MEETING ,MBTD106 1986, ANAHEIM US CAHN F. ET AL.: 'Porous microcarrier particles for mammalian cell culture' * Zusammenfassung * --- | 1,3-6 | C12N5/00 C12N5/06 |
| X | TRENDS IN BIOTECHNOLOGY. Bd. 8, Nr. 5, Mai 1990, CAMBRIDGE GB Seiten 131 - 136; CAHN F.: 'Biomaterials aspects of porous microcarriers for animal cell culture' * das ganze Dokument * --- | 1-6 | |
| X | BIOPHARM Bd. 3, Nr. 7, August 1990, Seiten 20 - 23; ADEMA E. ET AL.: 'Use of porous microcarriers in agitated cultures' * das ganze Dokument * --- | 1,3-6 | |
| X | WO-A-8 605 811 (VERAX CORPORATION) * Zusammenfassung; Ansprüche 1-13,27 * --- | 1-6 | **RECHERCHIERTE SACHGEBIETE (Int. Cl.5 )** C12N |
| A | DE-A-3 347 512 (HUMBOLDT-UNIVERSITÄT ZU BERLIN) * das ganze Dokument * --- | 1-6 | |
| A | DE-A-3 629 692 (HUMBOLDT-UNIVERSITÄT ZU BERLIN) * das ganze Dokument * --- | 1-6 | |
| A | WO-A-8 700 197 (KARYON TECHNOLOGY,INCORPORATED) * Ansprüche 1-5,12-13,19,20 * ------ | 1-6 | |

Der vorliegende Recherchenbericht wurde für alle Patentansprüche erstellt

| Recherchenort | Abschlußdatum der Recherche | Prüfer |
|---|---|---|
| BERLIN | 05 MAERZ 1992 | GURDJIAN D. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes
    Dokument

EPO FORM 1503 03.82 (P0400)